# EUROPEAN PATENT SPECIFICATION

(11) **EP 3 718 482 B1**
(45) Date of publication and mention of the grant of the patent: **25.08.2021**
(21) Application number: 20167772.1
(22) Date of filing: 02.04.2020
(51) Int. Cl.: A61B 8/00, A61G 15/02, A61B 8/08

(54) **ULTRASOUND DIAGNOSTIC APPARATUS AND CONTROL METHOD THEREOF**
ULTRASCHALLDIAGNOSEVORRICHTUNG UND STEUERUNGSVERFAHREN DAFÜR
APPAREIL DE DIAGNOSTIC ULTRASONIQUE ET PROCÉDÉ DE COMMANDE ASSOCIÉ

(30) Priority: 02.04.2019 KR 20190038284; 10.06.2019 KR 20190068021
(43) Date of publication of application: 07.10.2020
(73) Proprietor: SAMSUNG MEDISON CO., LTD., Hongcheon-gun, Gangwon-do (KR)
(72) Inventor: KANG, Hyewon, 16708 Gyeonggi-do (KR); SUNG, Giseok, 16807 Gyeonggi-do (KR); KIM, Yonghyun, 13475 Gyeonggi-do (KR); YANG, Choong-Mo, 16679 Gyeonggi-do (KR); LEE, Taeho, 18482 Gyeonggi-do (KR); SHIN, Sokjae, 16868 Gyeonggi-do (KR); JANG, Ku-Il, 17108 Gyeonggi-do (KR); HEO, Minho, 16307 Gyeonggi-do (KR)
(74) Representative: Arnold & Siedsma

(56) References cited:
- WO-A1-2018/056651
- US-A1- 2017 150 944
- US-A1- 2018 085 271

## Description

### BACKGROUND

### 1. Field

The disclosure relates to an ultrasound diagnostic apparatus in which a control board and a table for mounting a probe are separated, and a control method thereof.

### 2. Description of the Related Art

An ultrasound diagnostic apparatus irradiates an ultrasound signal generated from a transducer of a probe to an object, receives information of a signal reflected from the object, and obtains at least one image of a part (eg, soft tissue or blood flow) inside the object.

The ultrasound diagnostic apparatus is compact, inexpensive, non-invasive and non-destructive when compared to other imaging devices such as X-ray devices, CT scanners, MRI, nuclear medicine diagnostic devices, etc. and is widely used for diagnosis of gynecology, heart, abdomen and urology.

On the other hand, in the conventional ultrasound diagnostic apparatus, there is no table for mounting the probe, or the probe is provided to be mounted around the control board in which the user is located. Therefore, when the user selects the probe before diagnosis, there is no inconvenience that requires many moving line.

In addition or as an alternative for the above acknowledged prior art, reference is made here to US-2018/085271 and WO-2018/056651, relative to which the present disclosure is distinguished by features defined in the characterizing portions of appended independent claims.

### SUMMARY

Therefore, it is an aspect of the disclosure to provide an ultrasound diagnostic apparatus that may allow a user to easily hold the probe before diagnosis, may provide the convenience of diagnosis to the user during the diagnosis, and may facilitate the access of test subjects even after diagnosis by moving the table on which the probe is mounted according to the user's intention and diagnostic sequence, and a control method thereof.

In accordance with one aspect of the disclosure, an ultrasound diagnostic apparatus includes features defined in the appended independent device claim.

In accordance with another aspect of the disclosure, a control method of an ultrasound diagnostic apparatus including a table configured to mount at least one probe, wherein the method includes features defined in the appended independent method claim.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a perspective view illustrating an appearance of an ultrasound diagnostic apparatus according to an embodiment.
FIG. 2 is a view of the ultrasound diagnostic apparatus viewed from an X axis according to an embodiment.
FIG. 3 is a view of the ultrasound diagnostic apparatus viewed from the Y axis according to the embodiment.
FIG. 4 is a control block diagram of an ultrasound diagnostic apparatus according to an embodiment.
FIG. 5 is a view for illustrating an embodiment of a first sensor.
FIG. 6 is a view for illustrating an embodiment of the third sensor.
FIG. 7 is a flowchart illustrating a control method according to an embodiment, and FIG. 8 is a view for supplementing the embodiment of FIG. 7
FIG. 9 is a flowchart illustrating a control method according to another embodiment, and FIG. 10 is a view for supplementing the embodiment of FIG. 9.
FIG. 11 is a flowchart illustrating a control method according to another embodiment, and FIG. 12 is a view for supplementing the embodiment of FIG. 11.
FIG. 13 is a control block diagram of an ultrasound diagnostic apparatus according to another embodiment.
FIGS. 14A and 14B are views for illustrating a regression device according to different embodiments.
FIG. 15 is a flowchart for illustrating an operation of the ultrasound diagnostic apparatus according to another embodiment.
FIGS. 16 and 17 are views for illustrating the flowchart of FIG. 15 in detail.
FIG. 18 is a flow chart for illustrating the operation of the ultrasound diagnostic apparatus according to another embodiment.
FIGS. 19 and 20 are views for illustrating the flowchart of FIG. 18 in detail.

### DETAILED DESCRIPTION

Like reference numerals refer to like elements throughout the specification. Not all elements of embodiments of the disclosure will be described, and description of what are commonly known in the art or what overlap each other in the embodiments will be omitted. The terms as used throughout the specification, such as "~ part," "~ module," "~ member," "∼ block," etc., may be implemented in software and/or hardware, and a plurality of "∼ parts," "∼ modules," "~ members," or "∼ blocks" may be implemented in a single element, or a single "∼ part," "~ module," "~ member," or "∼ block" may include a plurality of elements.

It will be understood that when an element is referred to as being "connected" to another element, it can be directly or indirectly connected to the other element, wherein the indirect connection includes "connection" via a wireless communication network.

Also, when a part "includes" or "comprises" an element, unless there is a particular description contrary thereto, the part may further include other elements, not excluding the other elements.

Further, when it is stated that a layer is "on" another layer or substrate, the layer may be directly on another layer or substrate or a third layer may be disposed therebetween.

It will be understood that, although the terms first, second, third, etc., may be used herein to describe various elements, it should not be limited by these terms. These terms are only used to distinguish one element from another element.

As used herein, the singular forms "a," "an" and "the" are intended to include the plural forms as well, unless the context clearly indicates otherwise.

An identification code is used for the convenience of the description but is not intended to illustrate the order of each step. Each of the steps may be implemented in an order different from the illustrated order unless the context clearly indicates otherwise.

Hereinafter, the operation principles and embodiments of the disclosure will be described with reference to the accompanying drawings.

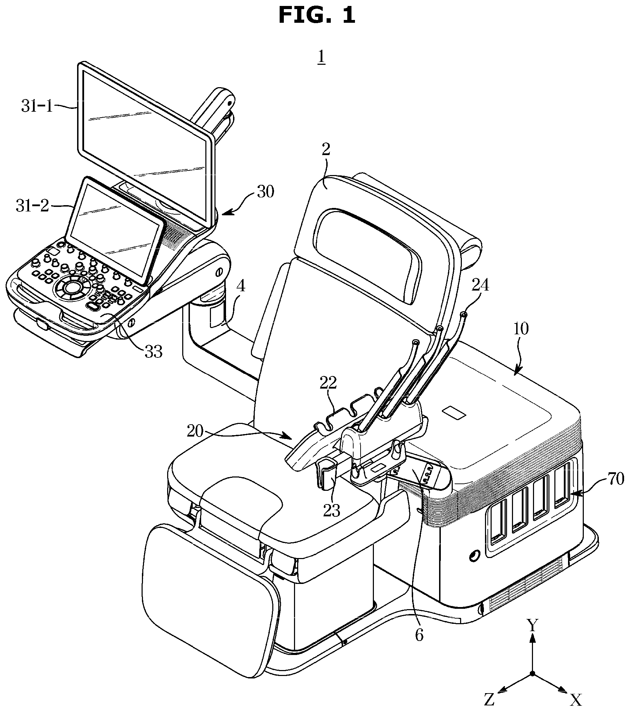

FIG. 1 is a perspective view illustrating an appearance of an ultrasound diagnostic apparatus according to an embodiment.

Referring to FIG. 1, the disclosed ultrasound diagnostic apparatus 1 includes a seat (2) in which an object may seat, a main body (10) provided in the Z-axis direction relative to the seat (2), a first arm (4) connected to one side of main body (10), a second arm (6) connected to the other side of the main body (10), a control panel 30 supported by first arm 4 and a table (20) supported by second arm (6).

The object may be a human or an animal having blood vessels, but is not limited thereto, and when the internal structure may be imaged by the ultrasound diagnostic apparatus 1, it may be an object.

After the object is seated, the seat 2 may be changed in shape by the user's input. In addition, the seat 2 may further include various components such as a weight sensor (not shown) for detecting whether the object is seated and transmitting the generated electrical signal to the main body 10.

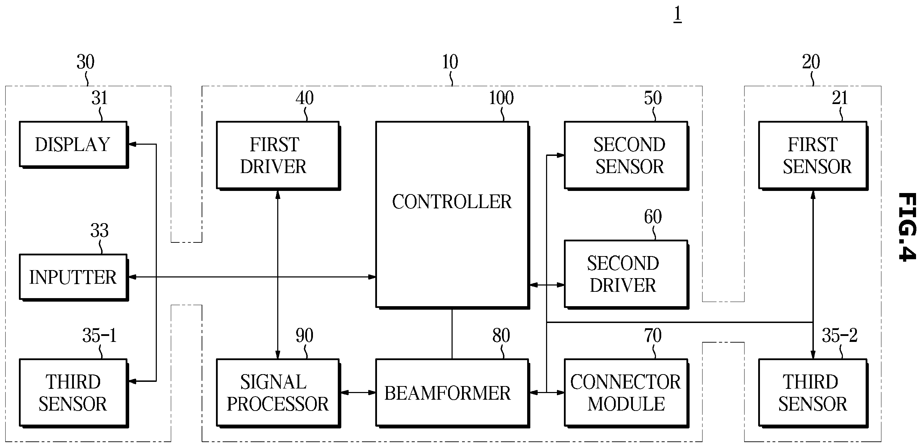

The main body 10 may be provided with various configurations necessary for the operation of the ultrasound diagnostic apparatus 1. For example, the main body 10 may include a connector module 70 which can be electrically connected to a probe P, a driver (40, 60 see FIG. 4) providing a driving force to the first arm (4) or / and the second arm (6) and a beam former 80 (see FIG. 4) for performing a time delay of the ultrasound signal transmitted to the object and received from the object by the probe P.

A detailed description of the various components included in the main body 10 will be described later with reference to FIG. 4.

The control panel 30 supported by the first arm 4 may include displays 31-1 and 31-2 and an inputter 33 for the user to control the ultrasound diagnostic apparatus 1. The displays 31-1 and 31-2 may display an ultrasound image and a user interface. The inputter 240 may receive various control commands such as an input command for controlling the ultrasound diagnostic apparatus 1 and a control command for the probe P, as well as an input command for instructing the movement of the table 20 from the user.

The table 20 supported by the second arm 6 includes a variety of configurations to mount at least one probe (P).

The table 20 includes a probe fixture 22 provided in the shape of a groove in which the probe P can be mounted by a cable ball. In addition, the table 20 may include a hook 24 to fix the cable and allow the user to move the probe P with little force.

The table 20 and the control panel 30 connected to the arms 4 and 6 are operated by the first driver 40 and the second driver 60 provided in the main body 10. In particular, the disclosed ultrasound diagnostic apparatus 1 moves the table 20 based on a user's input command, a change in the mounting state of the probe P, and the like. A detailed description of the moving direction and the moving distance of the table 20 will be described later with reference to the following drawings.

Meanwhile, the disclosed ultrasound diagnostic apparatus 1 may further include various configurations in addition to the above configurations.

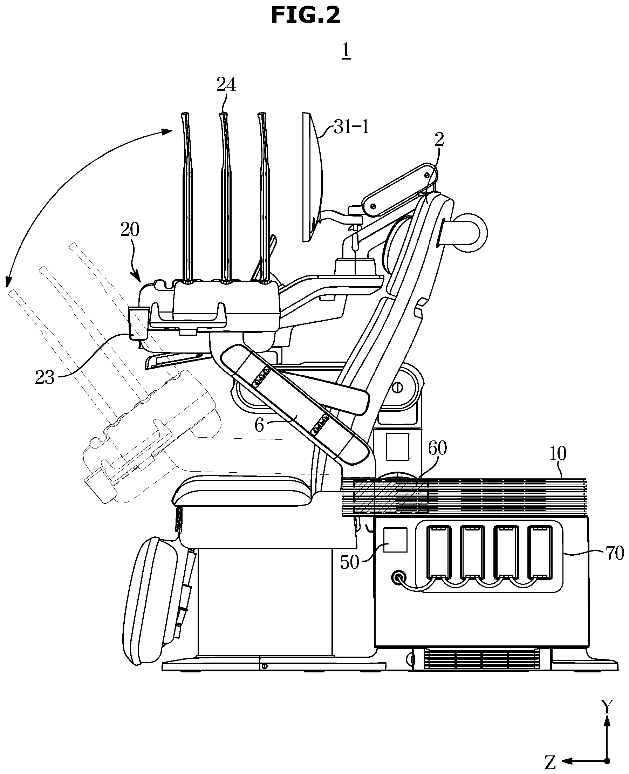

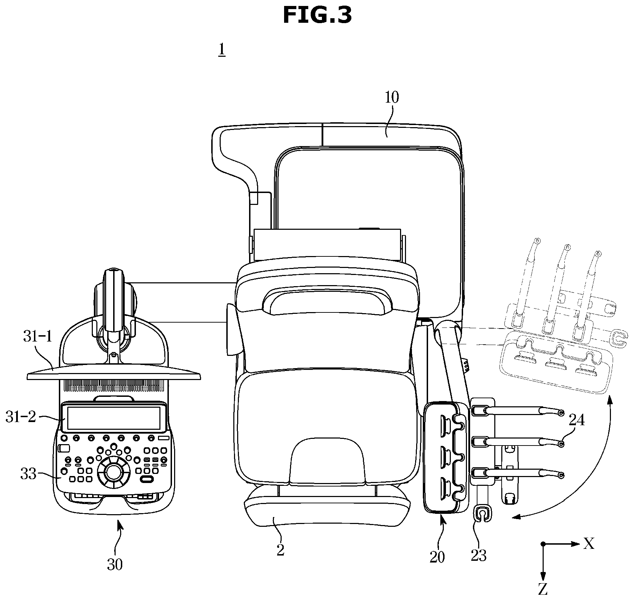

FIG. 2 is a view of the ultrasound diagnostic apparatus viewed from an X axis according to an embodiment. FIG. 3 is a view of the ultrasound diagnostic apparatus viewed from the Y axis according to the embodiment. In order to avoid overlapping description, it demonstrates together below.

According to an embodiment, the second arm 6 may be hinged to one side of the main body 10. The second arm 6 may rotate by being provided with a driving force by the driver 60.

Referring to FIG. 2, the second arm 6 may rotate based on the X axis through a driving force transmitted by the second driver 60 provided in the main body 10. Through this, the ultrasound diagnostic apparatus 1 may adjust the height of the table 20.

Referring to FIG. 3, the second arm 6 may rotate based on the Y axis through a driving force transmitted by the second driver 60 provided in the main body 10. Through this, the ultrasound diagnostic apparatus 1 may move the table 20 closer to the control panel 30 and may move the table 20 away from the control panel 30.

Meanwhile, when one side of the second arm 6 is hinged to the main body 10, the other side of the second arm 6 may be hinged to the table 20. By hinge coupling with the second arm (6), the table (20) may be rotated about the X axis. Therefore, unlike shown in FIG. 2, when the height of the table 20 is adjusted, the inclination of the table 20 may be adjusted to be parallel to the Z axis.

Inside the main body 10, a second sensor 50 capable of identifying the position of the table 20 may be provided. Based on the detection result of the second sensor 50, the ultrasound diagnostic apparatus 1 determines the position of the table 20 and performs the following control method operations.

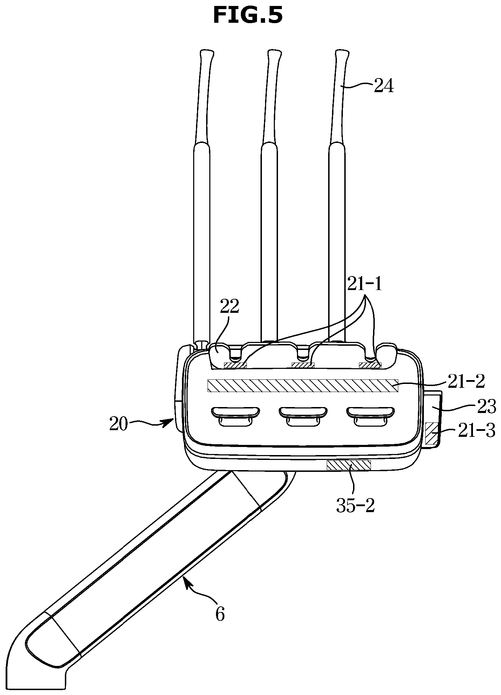

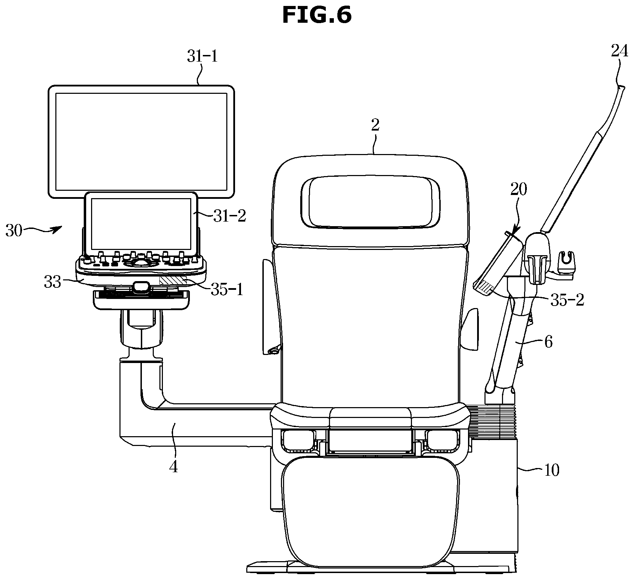

FIG. 4 is a control block diagram of an ultrasound diagnostic apparatus according to an embodiment. FIG. 5 is a view for illustrating an embodiment of a first sensor. FIG. 6 is a view for illustrating an embodiment of the third sensor.

Referring to FIG. 4, the ultrasound diagnostic apparatus 1 may include a main body 10 having various configurations such as a controller 100, a table 20 connected to the main body 10 through respective arms 4 and 6 and the control panel 30.

The main body 10 may include a first driver 40 driving the first arm 4 and a second driver 60 driving the second arm 6, a second sensor (50) for sensing the position of the table (20), a connector module 70 corresponding to the passage of the control signal for controlling the probe (P) and the ultrasound signal received from the probe (P), a beam former 80 for receiving an ultrasound signal from the connector module 70, a signal processor 90 for improving the image quality of the ultrasound image based on the signal transmitted from the beamformer 80 and a controller (100) for controlling the overall configuration of the ultrasound diagnostic apparatus (1).

The table 20 may include a first sensor 21 for detecting type information of the probe P and a mounting state of the probe P, and a third sensor 35-2 for detecting a gesture of a user.

The control panel 30 may include a display 31 for outputting an ultrasound image and an interface, an inputter 33 for receiving a user's input, and a third sensor 35-1 for receiving a user's gesture as provided in a table 20.

Specifically, the first sensor 21 may detect a change in the mounting state of the probe P and the type of the probe P.

When the user grabs one of the probes (P) mounted on the table (20), The first sensor 21 may detect a change in the mounting state of the probe P and the type of the probe P by detecting vibration or detachment of the probe P.

The first sensor 21 may be composed of a single or a plurality of sensors, and the sensor may be provided in at least one probe fixtures 22 provided in the table 20, or may be provided inside the table 20.

Referring to FIG. 5, a sensor 21-1 that detects a change regarding a mounting state of a probe P among first sensors according to an embodiment may be provided in the probe fixture 22. For example, the sensor 21-1 may determine the mounting state based on whether the cable ball is separated. Another sensor 21-2 for detecting a change in the mounting state among the first sensor may be provided inside the table 20. Sensor 21-3 according to another embodiment may be provided in the hook 23 capable of mounting the probe by inserting the probe (P).

The controller 100 may determine the type of the probe P by distinguishing the sensors of the first sensor 21 provided at different positions.

The sensor included in the first sensor 21 may include at least one of a magnet sensor, a metal sensor, or a radio frequency (RF) sensor.

The first sensor 21 converts the detected result into an electrical signal and transmits the result to the controller 100. The controller 100 may determine the changed probe P in the table 20 based on the received electrical signal. A specific embodiment of the position of the sensor of the first sensor 21 will be described later with reference to FIG. 5.

Referring back to FIG. 4, the second sensor 50 is provided inside the main body 10 as described above with reference to FIG. 2. The second sensor 50 may be configured as a sensor for detecting the position of the table 20. The second sensor 50 converts the detected signal into an electrical signal and transmits the detected signal to the controller 100.

For example, when the second sensor 50 is provided as a hall sensor, the second sensor 50 may determine the position of the table 20 based on a change in the magnetic field that is changed according to the position of the table 20. As another example, when the second sensor 50 is provided as a motor position sensor, the second sensor 50 may detect the position of the table 20 by measuring the rotation speed of the motor providing the driving force to the table 20.

The third sensors 35-1 and 35-2 detect the gesture of the user. The third sensors 35-1 and 35-2 may include a camera module for capturing a gesture of a user or an ultrasound sensor for detecting a gesture.

For example, when the third sensors 35-1 and 35-2 are provided as camera modules, the third sensors 35-1 and 35-2 may detect the user's gesture or distance from the user through the imaging analysis. As another example, when the third sensors 35-1 and 35-2 are provided as ultrasound sensors, the third sensors 35-1 and 35-2 may secure the three-dimensional detection area and detect the user's gesture or distance from the user by measuring the reflector reflected by the user.

The result of detection of the third sensors 35-1 and 35-2 is transmitted to the controller 100, and the controller 100 may determine the detected user's gesture as the user's input.

Referring to FIG. 6, third sensors 35-1 and 35-2 may be provided in the table 20 or the control panel 30. In detail, the third sensor 35-1 provided on the control panel 30 may be placed on one side of the inputter 33, and may detect a user's approach or a user's gesture.

The third sensor 35-2 provided in the table 20 may also detect whether the user approaches the control panel 30 or detect a specific gesture of the user. In order to secure a constant shooting range, the third sensor 35-2 may be provided at the lower end of the table 20.

Meanwhile, the position of the sensor described above with reference to FIGS. 5 and 6 is merely an example. That is, the sensor may be provided at various positions for the result to be measured, and may be changed. The disclosed ultrasound diagnostic apparatus 1 may further include various sensors in addition to the above-described sensors.

Referring back to FIG. 4, the display 31 may display an ultrasound image of a target area inside the object. The ultrasound image displayed on the display 31 may be a 2D ultrasound image or a 3D stereoscopic ultrasound image, and various ultrasound images may be displayed according to an operation mode of the ultrasound diagnostic apparatus 1.

The display 31 may display not only menus or guidance items necessary for ultrasound diagnosis, but also information on an operation state of the probe P through various user interfaces. In detail, the display 31 may output whether or not the mounting state of the probe P changed in the table 20 is changed through the user interface, and the type of probe (P) whose mounting state has changed and the type and number of probes (P) currently mounted on the table (20) may also be displayed. In addition, the display 31 may output the current position of the table 20 and the control panel 30 to be described later and the table (20) and the control panel (30) where the movement is completed under the control of the driver (40, 60) through the user interface.

The display 31 may be divided into a first display 31-1 and a second display 31-2. The first display 31-1 outputs not only menus or guidance items necessary for the above-described ultrasound image and ultrasound diagnosis, but also displays such as information on an operation state of the probe 100. And the second display 31-2 may provide related information such as a menu or an auxiliary image for optimizing an ultrasound image, or may provide various user interfaces described above. When the second display 31-2 serves as an inputter 33 to be described later, the second display 31-2 may display a graphic user interface having the same shape as a button included in the input device 33.

The display 31 may be implemented by various known methods, such as a cathode ray tube (CRT), a liquid crystal display (LCD), a light emitting diode (LED), a plasma display panel (PDP), an organic light emitting diode (OLED), or the like.

The inputter 33 receives a user's input command.

The input command received by the inputter 33 may include various input commands for executing the operation of the ultrasound diagnostic apparatus 1, such as a command regarding movement of the table 20 and the control panel 30, an input command regarding ultrasound diagnosis, and the like.

The inputter 33 may be implemented as various hardware devices such as a keyboard, a foot switch, or a foot pedal. For example, when the inputter 33 is implemented as a keyboard, the keyboard may include at least one of a switch, a key, a joystick, and a trackball.

On the other hand, the inputter 33 is not necessarily provided in the control panel 30. For example, when the inputter 33 is provided with a hardware device such as a foot switch or a foot pedal, the inputter 33 may be provided under the seat 2 or the main body 10.

The first driver 40 and the second driver 60 drive the arms 4 and 6 connected to the main body 10. The first driver 40 and the second driver 60 may include a motor for producing a driving force, and the controller 100 may further include various modules such as an inverter for controlling the motor.

The connector module 70 includes a connector (for example, a male / female connector) corresponding to the connector of one end of the cable connected with the probe (P) and may include various configurations such as a printed circuit board (PCB), which is a connection path for ultrasound signals received through a probe (P) or various control signals, and a relay for selecting one probe among the plurality of probes (P). The connector module 70 may be provided on one surface of the main body 10.

The beam former 80 allows the ultrasound generated from the transducer (not shown) of the probe P to be focused at one target point of the object at the same time desired, or may provide an appropriate time delay to the irradiated ultrasound or the received echo ultrasound to overcome the time difference at which echo ultrasound reflected from one target point of the object reaches the transducer.

The signal processor 90 filters the noise components in the received digital focused beam to improve the image quality of the ultrasound image, performs an envelope detection process for detecting the strength of the received signal based on the filtered received focused beam and generates a digital ultrasound image data.

The signal processor 90 may perform a scan conversion for converting the scanning lines of the digital ultrasound image data so that the digital ultrasound image data can be displayed on the display 31 and perform image processing such as B mode image processing, Doppler image processing, etc. on the digital ultrasound image data to display an ultrasound image of a desired shape based on the scan-converted digital ultrasound image data.

The signal processor 90 performs RGB processing on the ultrasound image data and transmits the ultrasound image data to the display 31 so that the image-processed digital ultrasound image data can be displayed as an ultrasound image.

The controller 100 is a configuration that controls the ultrasound diagnostic apparatus 1, and is a processor that collectively controls various configurations provided in a main body 10, a table 20, and a control panel 30.

In particular, the controller 100 may move the table 20 to a proximal position of the user so that the user may catch a plurality of probes P mounted on the table 20. The controller (100) may use the detection result of the second sensor 50 to detect the current position of the table 20 and the position to be moved for the movement of the table 20 and may use detection results of third sensors 35-1 and 35-2 to sense a user's position.

In addition, when the user changes the mounting state of the plurality of probes P mounted on the table 20, the controller 100 moves the table 20 again for the convenience of diagnosis. The controller (100) may use the detection result of the first sensor 21 to detect a change in state concerning the mounting of the probe P and may use the detection result of the second sensor 50 to determine the current position of the table 20 and the position to move to.

When the diagnosis is completed, such as the end of the system or the end of the photographing sequence, the controller 100 may move the table 20 away from the seat 2 to facilitate the movement of the object. The controller (100) may use detection results of third sensors 35-1 and 35-2 for receiving input related to the end of photographing and the like, and may use the detection result of the second sensor 50 to determine the current position of the table 20 and the position to move to.

In addition, the controller 100 may perform various functions for general operation of the ultrasound diagnostic apparatus 1. For example, the controller 100 may control the probe P to form an ultrasound signal to be applied to each of the plurality of transducers in consideration of the positions and focal points of the plurality of transducers included in the probe P selected by the user.

The controller 100 may receive the echo ultrasound signal reflected by the object from the probe P, and then display the ultrasound image generated by the beamformer 80 and the signal processor 90 on the display 31.

The controller 100 may include a ROM in which a control program for controlling the ultrasound diagnostic apparatus 1 is stored and a RAM used as a storage area corresponding to various operations performed in the ultrasound diagnostic apparatus 1. In addition, the controller 100 may be implemented as a processing board including the above-described processor, RAM, or ROM on a circuit board, and the processor, RAM, and ROM may be interconnected through an internal bus.

Meanwhile, the disclosed ultrasound diagnostic apparatus 1 may further include other components not described in FIG. 4, and the mutual positions of the components may be changed in correspondence with the performance or structure of the system.

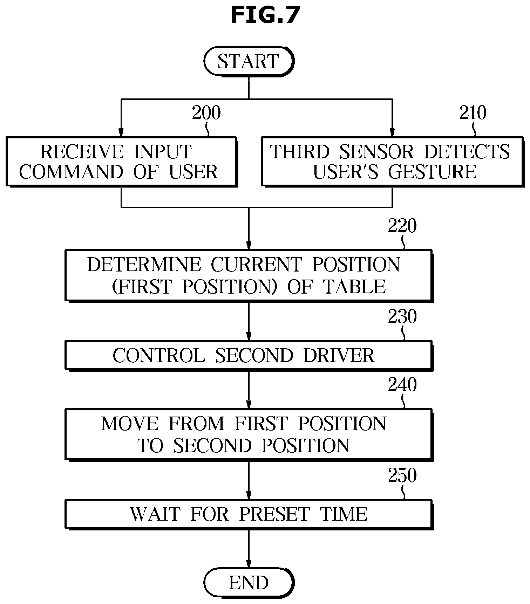

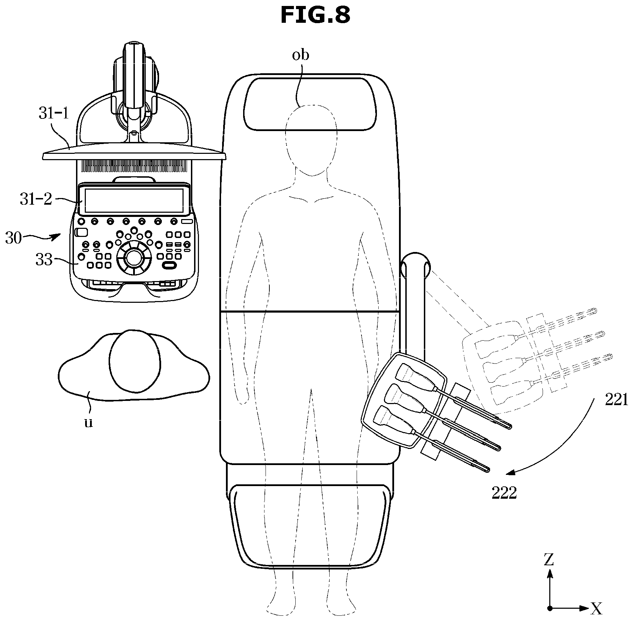

FIG. 7 is a flowchart illustrating a control method according to an embodiment, and FIG. 8 is a view for supplementing the embodiment of FIG. 7

Referring to FIGS.7 and 8 together, the ultrasound diagnostic apparatus 1 may receive an input command of the user (200), or the third sensors 35-1 and 35-2 may detect the user's gesture (210).

As shown in FIG. 8, the user U may access the control panel 30 and transmit an input command through the inputter 33. The gesture of the user may be received according to the detection result of the third sensors 35-1 and 35-2. The input command or the user gesture may be a signal for starting diagnosis of the object Ob.

The ultrasound diagnostic apparatus 1 determines a first position of the table (220).

The ultrasound diagnostic apparatus 1 determines the current position of the table 20 through the second sensor 50.

As shown in FIG. 8, the first position 221 may be a position where the ultrasound diagnostic apparatus 1 moves the table 20 in the previous diagnosis. However, the first position 221 is not necessarily designated, and the ultrasound diagnostic apparatus 1 determines the current position for the movement of the table 20.

The ultrasound diagnostic apparatus 1 controls the second driver 60 to move the position of the table 20 (230), and the table 20 moves from the first position 221 to the second position 222 (240).

In this case, the second position 222 is set in advance, and if the position of the table 20 is a position where the user U can catch the probe P mounted on the table 20, the position is sufficient as the second position 222.

FIG. 8 is a view of the ultrasound diagnostic apparatus 1 looking down from the Y axis. Therefore, the table 20 is illustrated as an example of rotating around the Y axis. However, the position where the table 20 moves may also include the height in the Y-axis direction.

The ultrasound diagnostic apparatus 1 moves the table 20 to the second position 222 and then waits for a preset time(250).

Through This, the ultrasound diagnostic apparatus 1 allows the user U to have enough time to select the probe P.

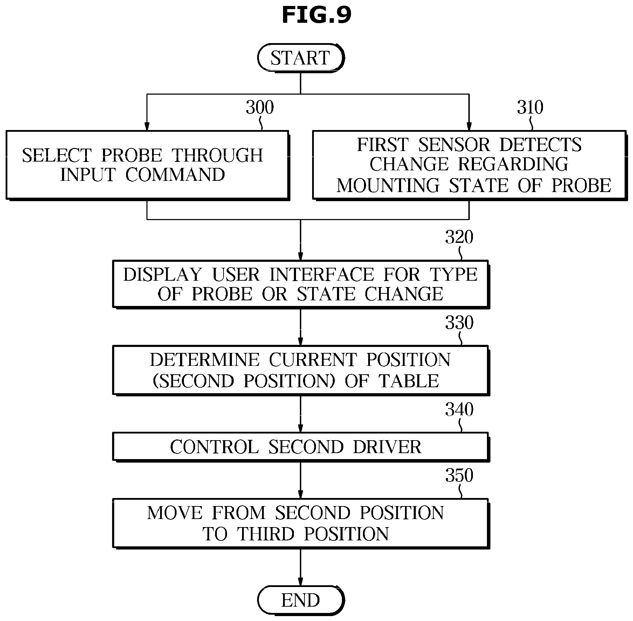

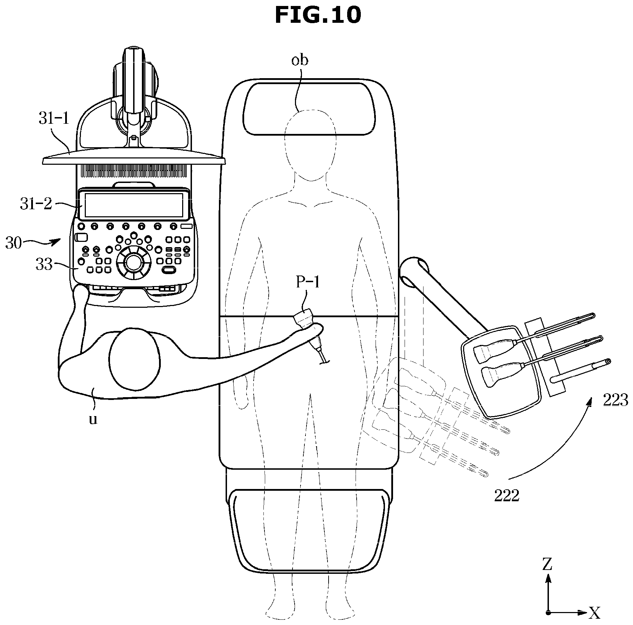

FIG. 9 is a flowchart illustrating a control method according to another embodiment, and FIG. 10 is a view for supplementing the embodiment of FIG. 9.

Referring to FIGS. 9 and 10 together, the user selects a probe 300 through the inputter 33 (300) or The ultrasound diagnostic apparatus 1 may detect a mounting of the probe through the first sensor 21 (310).

That is, as described above in the embodiment of FIG. 7, the ultrasound diagnostic apparatus 1 waits for the probe selection of the user U during the waiting time. During the waiting time, the ultrasound diagnostic apparatus 1 monitors the user U's probe selection.

As shown in FIG. 10, an input command may be received via an inputter 33. The input command may include information about the type of the selected probe (P-1). Also, the ultrasound diagnostic apparatus 1 may detect information about the type of probe and the change regarding the mounting state of the selected probe P-1 through the first sensor 21 without the input of the user U.

The ultrasound diagnostic apparatus 1 displays a user interface for a type of a probe or state change of a probe (320).

The user interface displayed here is determined from the input command of the user or the detection result of the first sensor 21.

The ultrasound diagnostic apparatus 1 determines the current position of the table 20 through the second sensor 50.

As shown in FIG. 10, the second position 222 may be a position where the ultrasound diagnostic apparatus 1 moves the table 20 for the convenience of the user U. However, the second position 222 is not necessarily designated, and the ultrasound diagnostic apparatus 1 may determine the current position to move the table 20.

The ultrasound diagnostic apparatus 1 controls the second driver 60 to move the position of the table 20 (340), the table 20 moves from the second position 222 to the second position 223 (350).

Here, the third position 223 is set in advance, and there is no limitation as long as the user U can avoid interference due to the position of the table 20 when the object Ob is diagnosed.

Meanwhile, in FIG. 10, the table 20 may move in the Y-axis direction similarly to FIG. 8.

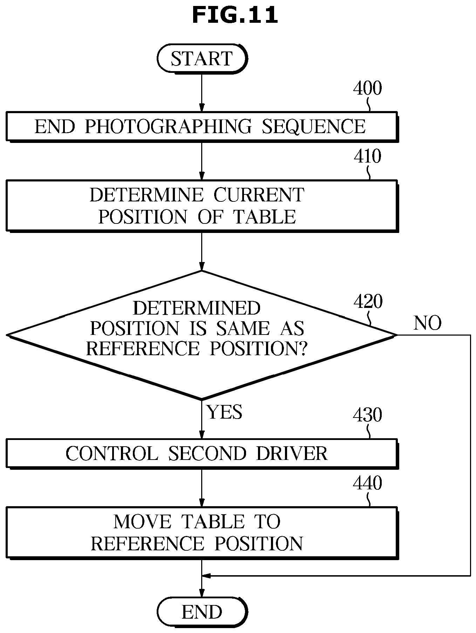

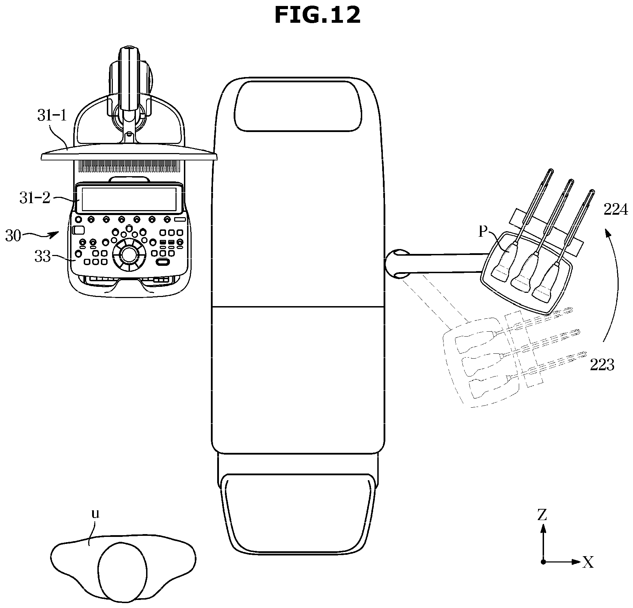

FIG. 11 is a flowchart illustrating a control method according to another embodiment, and FIG. 12 is a view for supplementing the embodiment of FIG. 11.

Referring to FIGS. 11 and 12 together, the user may end the photographing sequence (400).

Here, the end of the photographing sequence includes a case in which the user no longer performs ultrasound diagnosis, such as the end of diagnosis. The ultrasound diagnostic apparatus 1 may determine the end of the photographing sequence through an input command or a user gesture of the user U.

The ultrasound diagnostic apparatus 1 determines the current position of the table 20 through the second sensor 50.

The current position of the table 20 may be a state in which the user P mounts the probe P used in the diagnosis on the table 20 again. Therefore, the current position of the table 20 may not necessarily be the third position 223 or may be the second position 222.

The ultrasound diagnostic apparatus 1 compares the current position of the determined table 20 with a reference position (420).

In this case, the reference position may be a position where the table 20 is farthest from the seat, and may be variously set.

When the determined current position of the table 20 and the reference position match, the ultrasound diagnostic apparatus 1 does not move the table 20 further.

However, when the determined current position of the table 20 and the reference position do not match, the ultrasound diagnostic apparatus 1 controls the second driver 60 (430), and moves the table 20 to the reference position (440).

Through this, The disclosed ultrasound diagnostic apparatus allows the user to easily hold the probe before diagnosis by moving the table on which the probe is mounted according to the user's intention and diagnostic sequence.

In addition, the ultrasound diagnostic apparatus may provide the convenience of diagnosis to the user during diagnosis by selecting the probe and moving the table again, and may facilitate the subject's access to the seat by moving the table after the diagnosis is completed.

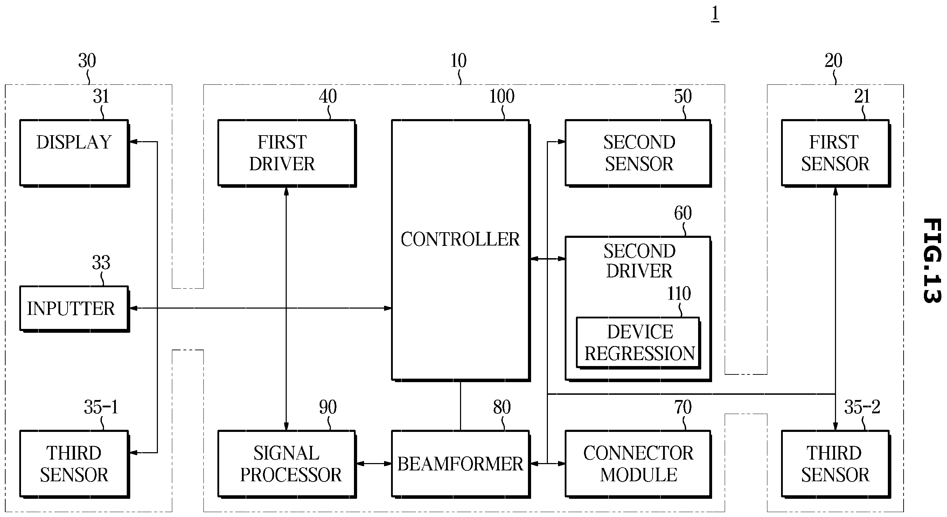

FIG. 13 is a control block diagram of an ultrasound diagnostic apparatus according to another embodiment.

Referring to FIG. 13, the ultrasound diagnostic apparatus 1 according to another embodiment may include a main body 10 having various configurations, a table 20 connected to the main body 10 through respective arms 4 and 6 and the control panel 30.

The main body 10 of the ultrasound diagnostic apparatus 1 may include a first driver 40 driving the first arm 4 and a second driver 60 driving the second arm 6, a second sensor (50) for sensing the position of the table (20), a connector module 70 corresponding to the passage of the control signal for controlling the probe (P) and the ultrasound signal received from the probe (P), a beam former 80 for receiving an ultrasound signal from the connector module 70, a signal processor 90 for improving the image quality of the ultrasound image based on the signal transmitted from the beamformer 80 and a controller (100) for controlling the overall configuration of the ultrasound diagnostic apparatus (1).

Unlike the case described with reference to FIG. 4, the ultrasound diagnostic apparatus 1 may further include a regression device 110 in the second driver 60. When the second arm 6 is moved by a force applied by the user or the like to the second arm 6 (hereinafter, external force), the regression device 110 moves the second arm 6 to a position before external force is applied again by the power means.

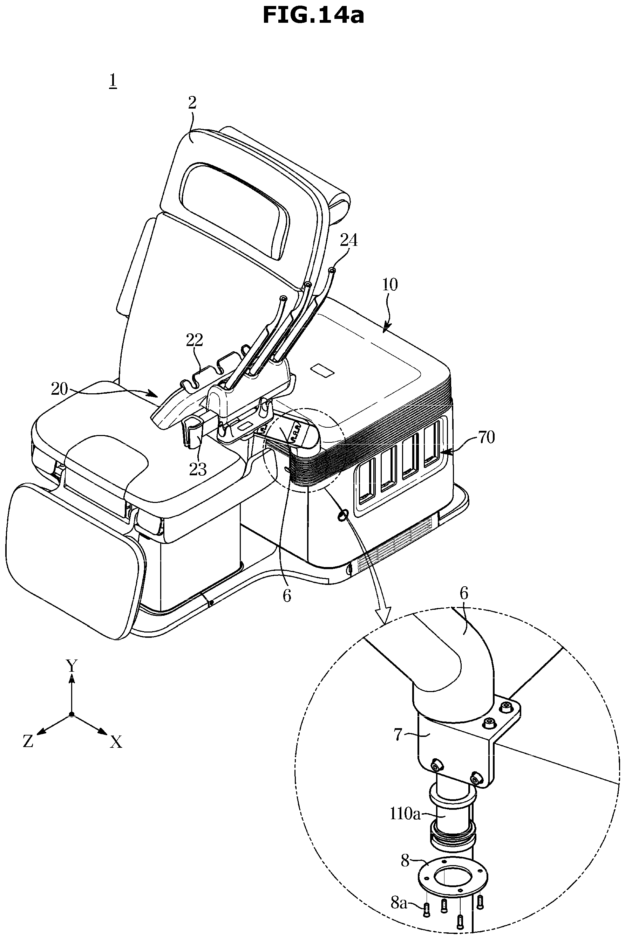

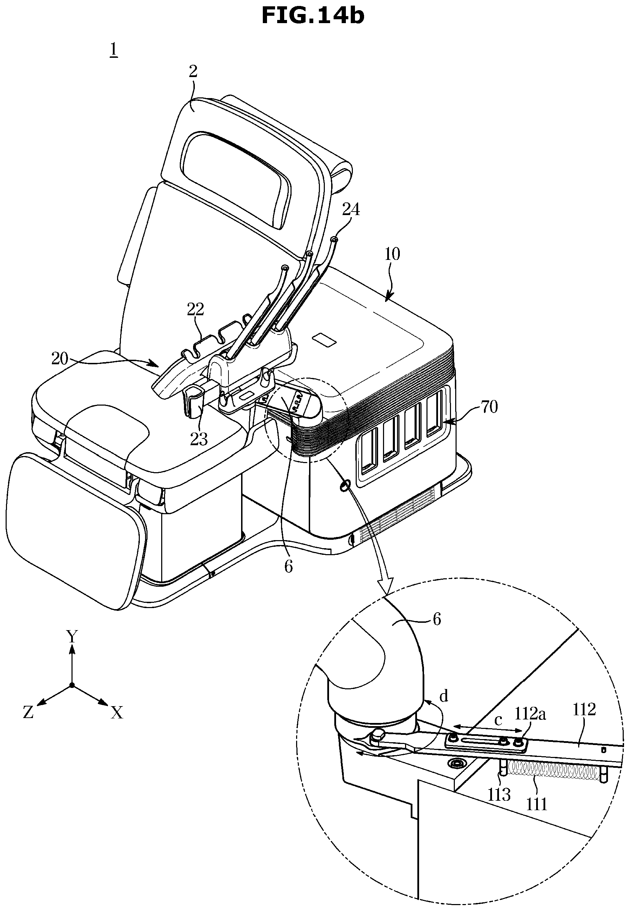

For example, the regression device 110 uses an elastic force of a torsion spring or a tension spring, and may select an inflection point of the movement of the table 20 based on the tension adjustment or the fixed shaft adjustment of the tension spring. A specific embodiment of the regression device 110 will be described later with reference to FIGS. 14A and 14B.

On the other hand, description of the overlapping configuration with FIG. 4 in the ultrasound diagnostic apparatus 1 according to another embodiment will be omitted.

FIGS. 14A and 14B are views for illustrating a regression device according to different embodiments. Specifically, FIG. 14A is a schematic diagram of an exploded perspective view of a regression device 110a including a torsion spring, FIG. 14B schematically illustrates an internal configuration of a regression device 110b including a tension spring.

First, referring to FIG. 14A, the regression device 110a may be provided inside the housing 7 connecting the second arm 6 and the main body 10. In addition, the regression device 110a inserted into the housing 7 may be supported by the bracket 8 and the pin 8a.

Although not specifically illustrated, the regression device 110a according to an embodiment may be divided into an shaft rotation unit (not shown) for transmitting torque generated by the torsional stress of the torsion spring, and a tension adjustment unit (not shown) fixing the revolution of the torsion spring. In addition, the regression device 110a may further include a stopper for limiting the rotation angle range of the table 20 and a detent for imparting a threshold resistance so that the table 20 rotated by the shaft rotation unit may stop at a specific position.

The regression device 110a may move the table 20 to a position before the external force is applied (hereinafter referred to as a fourth position) by the torque transmitted from the shaft rotation unit and may move table 20 to the fourth position for a preset time such as 5 seconds to 10 seconds.

Referring to FIG. 14B, a regression device 110b according to another embodiment may include a tension spring (111), a tension spring fixed shaft 113 to which the tension spring 111 is connected and a link bar 112 including a slot 112a for converting the linear motion c of the tension spring fixed shaft 113 by the elastic energy of the tension spring 111 into the rotational motion d of the second arm 6.

The regression device 110b applies the principle of conversion of the regression force action direction based on a preset angle. Through this, the regression device 110b may move the table 20 rotated beyond the preset angle to a reference position instead of the fourth position. Detailed description thereof will be described later with reference to other drawings.

Meanwhile, the regression device 110b may further include other components that are not described, such as a stopper, such as the regression device 110a according to an embodiment.

In addition to the devices described with reference to FIGS. 14A and 14B, the regression device 110 may move the table 20 through various devices, and is not necessarily limited to devices using elastic force such as torsion springs or tension springs.

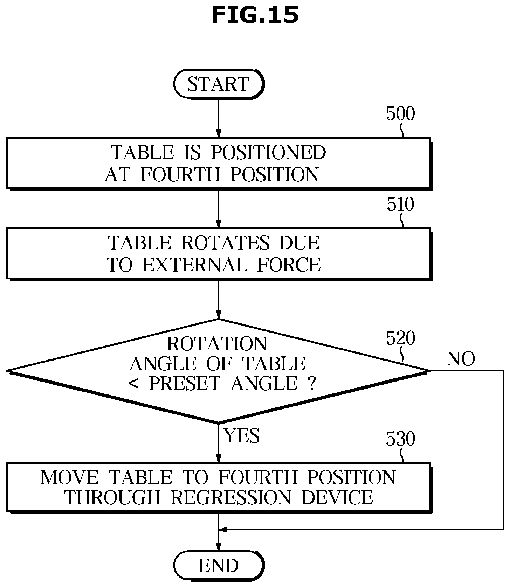

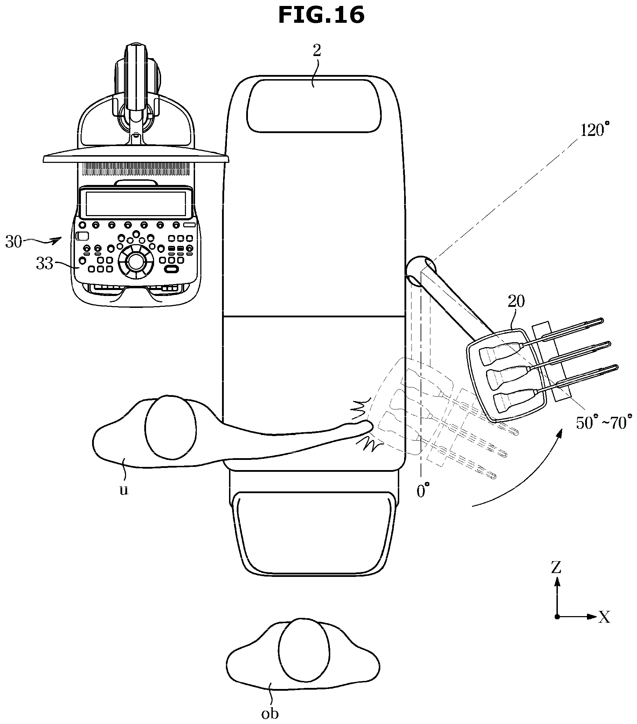

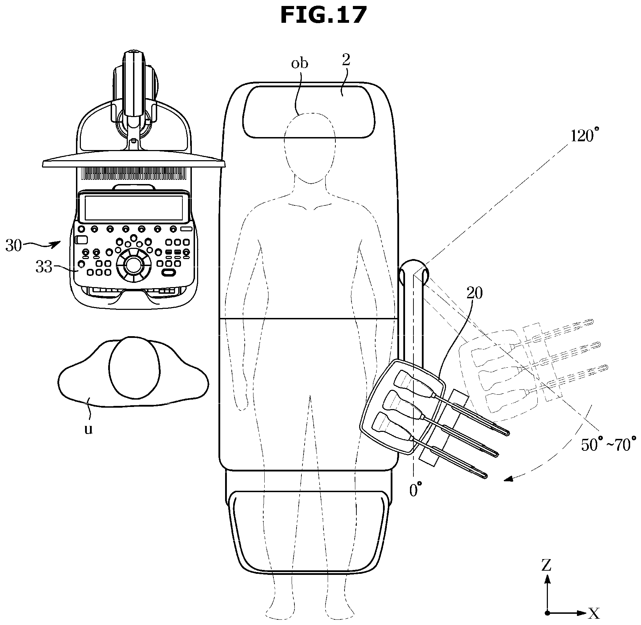

FIG. 15 is a flowchart for illustrating an operation of the ultrasound diagnostic apparatus according to another embodiment. FIGS. 16 and 17 are views for illustrating the flowchart of FIG. 15 in detail.

Referring to FIG. 15, the table 20 of the ultrasound diagnostic apparatus 1 is positioned at the fourth position (500).

Here, the fourth position means the rotation angle and the height of the table 20 positioned at 0° shown in FIGS. 16 and 17. The fourth position may be variously changed by setting of the second driver 60, for example, the regression device 110.

The table 20 rotates due to external force (510).

Referring to FIG. 16, as the user U enters the ultrasound diagnostic apparatus 1 together with the object Ob, the user U may push the table 20 that may interfere with the object Ob to be seated in the seat 2. In the same embodiment as in FIG. 16, the table 20 rotates counterclockwise with respect to the Y axis.

The ultrasound diagnostic apparatus 1 compares the rotation angle of the table 20 with a preset angle (520). If the rotation angle of the table 20 rotated by the external force is less than the preset angle, the ultrasound diagnostic apparatus 1 moves the table 20 to the fourth position through the regression device 110 (530).

Referring to FIG. 17, the external force applied by the user may rotate the table 20 to have a rotation angle smaller than 50 ° to 70 ° based on the fourth position. In this case, the ultrasound diagnostic apparatus 1 may rotate the table 20 back to 0 °. In addition, the ultrasound diagnostic apparatus 1 may control the time to move to 0 ° to have a preset time. This allows the ultrasound diagnostic apparatus 1 to provide sufficient time for the object Ob to sit on the seat 2.

The preset time may be, for example, 5 seconds to 10 seconds, but is not limited thereto. The preset time may vary through the setting of the regression device 110 or the second driver 60 controlled by the controller 100.

Meanwhile, the preset angle, which is a reference for moving the table 20 to the fourth position, may be variously changed.

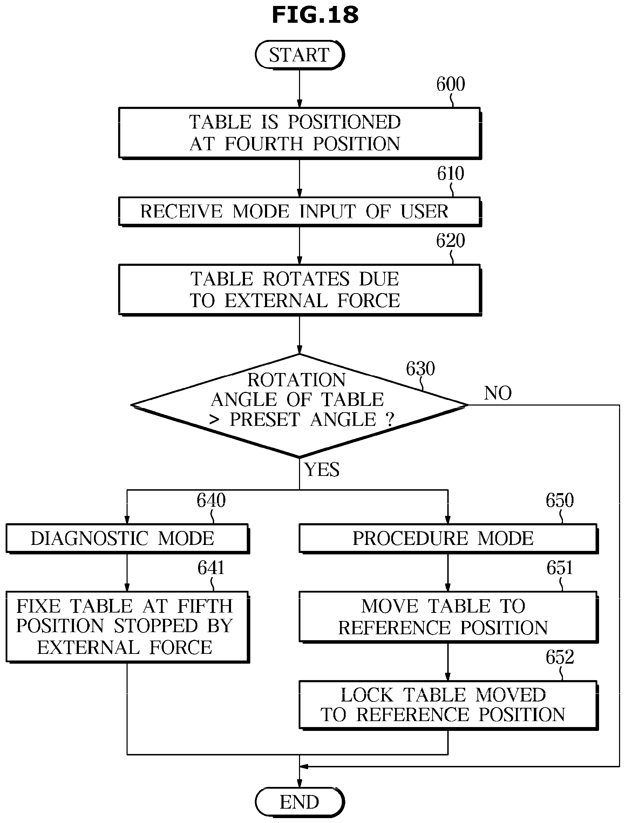

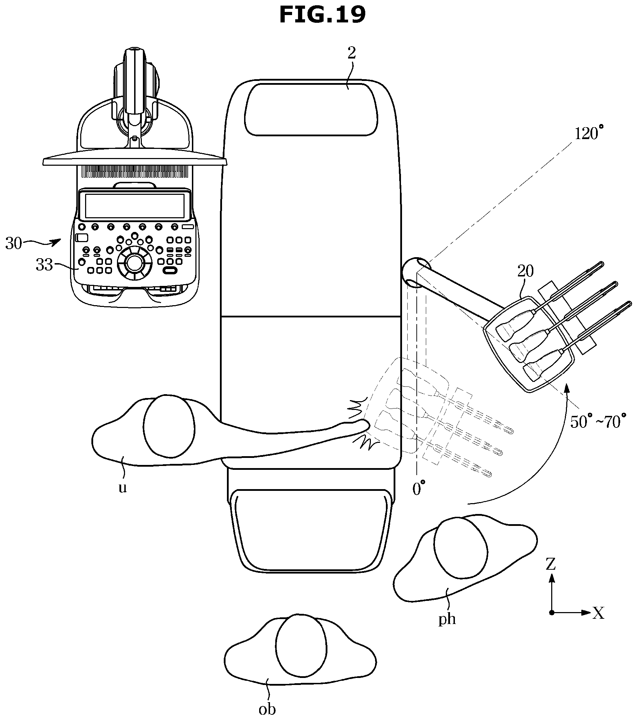

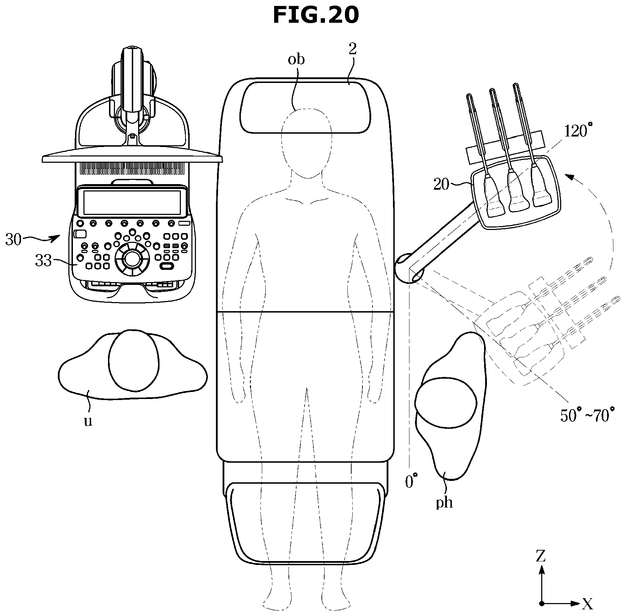

FIG. 18 is a flow chart for illustrating the operation of the ultrasound diagnostic apparatus according to another embodiment. FIGS. 19 and 20 are views for illustrating the flowchart of FIG. 18 in detail.

Referring to FIG. 18, the table 20 of the ultrasound diagnostic apparatus 1 is positioned at the fourth position (600).

Here, the fourth position means the rotation angle and height of the table 20 located at 0 ° shown in FIGS. 19 and 20. The fourth position may be variously changed by setting of the second driver 60, for example, the regression device 110.

The ultrasound diagnostic apparatus 1 receives a mode input of the user U (610).

In detail, the inputter 33 receives at least one of a diagnostic mode and a procedure mode. In this case, the diagnostic mode refers to an operation mode of a general ultrasound diagnostic apparatus in which a sonographer examines an object Ob using a probe P. The procedure mode refers to an operation mode of the ultrasound diagnostic apparatus in which the physician (Ph) together with the user U diagnoses the object Ob. The physician may use a sterile article including a syringe, needle or disinfecting cotton.

On the other hand, the input related to the mode received by the inputter 33 is converted into an electrical signal and transmitted to the controller (100).

Table 20 rotates due to external force (620).

Referring to FIG. 19, as the user U or the physician Ph enters the ultrasound diagnostic apparatus 1 together with the object Ob, and the user U or the physician Ph may push the table 20 that may interfere with the object Ob and the physician Ph. In the same embodiment as in FIG. 19, the table 20 rotates in a counterclockwise direction with respect to the Y axis.

Meanwhile, in the flowchart of FIG. 18, steps 610 and 620 are not necessarily performed in order. For example, the ultrasound diagnostic apparatus 1 may be set to a diagnostic mode as an initial setting, and the user U may perform mode input through the inputter 33 when the physician Ph needs to be entered during the examination.

The ultrasound diagnostic apparatus 1 compares the rotation angle of the table 20 with a preset angle (630). If the rotation angle of the table 20 rotated by the external force is greater than or equal to the preset angle, the ultrasound diagnostic apparatus 1 performs different control of the table 20 through the regression device 110 according to the received mode.

Specifically, in the diagnostic mode (640), the ultrasound diagnostic apparatus 1 compares the rotation angle of the table 20 rotated by the external force of the user with a preset angle, and fixes the table 20 at a position of the table 20 stopped by an external force, that is, at a fifth position (641).

That is, unlike the embodiment of FIG. 15, the ultrasound diagnostic apparatus 1 does not return the rotated table 20 in the diagnostic mode, and does not return the table 20 rotated more than a preset reference angle.

The preset angle may be 50 ° to 70 ° based on the fourth position as in FIG. 15, and may be variously changed.

In the procedure mode (650), the ultrasound diagnostic apparatus 1 compares the rotation angle of the table 20 rotated by the external force of the user with a preset angle, and moves the table 20 to a reference position from a fifth position of the table 20 stopped by an external force (651).

Referring to FIG. 20, the table 20 rotated by a predetermined angle or more by an external force may move to a reference position corresponding to 120 ° based on the fourth position through the second driver 60. As an example, the table 20 may move the table 20 stopped at the fifth position to the reference position by the principle of conversion of the regression force action direction of the regression device 110b described with reference to FIG. 14B. However, the disclosed embodiment is not necessarily limited thereto, and the table 20 may be moved to the reference position by the power generated by the second driver 60.

The ultrasound diagnostic apparatus 1 locks the table 20 moved to the reference position (652).

In detail, the controller 100 may fix the table 20 at a reference position by controlling a physical locking device included in the second driver 60. Through this, the ultrasound diagnostic apparatus 1 suppresses further movement of the table 20 so that spatial constraints do not occur in the behavior of the physician Ph in the procedure mode.

The ultrasound diagnostic apparatus and a control method thereof according to an aspect of the disclosure allow the user to easily hold the probe before diagnosis by moving the table on which the probe is mounted according to the user's intention and the diagnosis sequence.

The ultrasound diagnostic apparatus and a control method thereof according to another aspect of the disclosure may provide convenience of diagnosis to a user during diagnosis by moving a table again after selecting a probe.

The ultrasound diagnostic apparatus and a control method thereof according to another aspect of the disclosure may facilitate a test subject's access to a seat by moving a table after diagnosis is completed.

## Claims

1. An ultrasound diagnostic apparatus comprising:
a table (20) configured to mount at least one probe (P);
a first sensor (21) configured to detect a type information of the probe and a mounting state of the probe;
a driver (40) configured to generate a driving force for moving the table; and
a controller (100),
**CHARACTERIZED BY**
a second sensor (50) configured to detect a position of the table;
a third sensor (35) configured to detect a gesture of the user; and
wherein the controller (100) is configured to control the driver (40) based on detection results of the first sensor and the second sensor, and to determine a first position of the table (20) based on a detection result of the second sensor (50) and move the table (20) from the first position to a second position based on a detection result of the third sensor (35).

2. The ultrasound diagnostic apparatus according to claim 1, further comprising:
a display (31) configured to output an ultrasound image and an interface,
wherein the controller (100) is configured to determine whether or not the mounting state of the probe (P) mounted on the table (20) is changed based on a detection result of the first sensor (21) and display the mounting state and a type information of the probe whose mounting state has been changed on the display (31).

3. The ultrasound diagnostic apparatus according to claim 2, wherein the controller (100) is configured to stop moving the table (20) for a preset time after the mounting state of the probe (P) is changed and move the table (20) from the second position to a third position based on the preset time and the detection result of the first sensor (21).

4. The ultrasound diagnostic apparatus according to claim 1, wherein the controller (100) is configured to determine the position of the table (20) through the second sensor (50) based on a user input command regarding the end of the imaging sequence and move the table (20) from the determined position of the table to a reference position.

5. The ultrasound diagnostic apparatus according to claim 1, wherein the first sensor (21) comprises at least one probe fixture provided in the table (20) or at least one of a magnetic sensor, a metal sensor or an RF sensor provided inside the table.

6. The ultrasound diagnostic apparatus according to claim 1, further comprising:
a main body (10) comprising a beam former (80) and a connector (70) connected to the at least one probe (P);
a first arm (4) configured to support an inputter configured to receive a user input command and hinged to one side of the main body to be rotatable; and
a second arm (6) configured to support the table (20), hinged to the other side of the main body and rotatably coupled with respect to a first reference axis and a second reference axis,
wherein the controller (100) is configured to adjust the radius of rotation of the second arm (6) by adjusting the driver (40).

7. The ultrasound diagnostic apparatus according to claim 6, wherein the second sensor (50) is provided in the main body (10) and comprises at least one of a hall sensor or a motor position sensor,
wherein the third sensor (35) is provided inside the table (20) or on the inputter configured to receive a user input command and comprises at least one of a camera module or an ultrasound sensor.

8. A control method of an ultrasound diagnostic apparatus comprising a table (20) configured to mount at least one probe (P), comprising;
detecting a type information of the probe and a mounting state of the probe (P) by a first sensor (21);
detecting a position of the table (20) by a second sensor (50);
detecting a gesture of a user by a third sensor (35);
determining a first position of the table (20) based on a detection result of the second sensor (50); and
moving the table (20) based on a change of the detected mounting state and a position of the table,
wherein the moving comprises:
moving the table (20) from the first position to a second position based on the gesture of the user.

9. The control method according to claim 8, further comprising:
determining whether or not the mounting state of the probe (P) is changed after the table (20) is moved to the second position; and
displaying the mounting state and a type information of the probe (P), whose mounting state has been changed on a display (31).

10. The control method according to claim 8, further comprising:
stopping moving the table (20) for a preset time after the mounting state of the probe (P) is changed,
wherein the moving comprises:
moving the table (20) from the second position to a third position based on the preset time and a change in the mounting state of the probe (P).

11. The ultrasound diagnostic apparatus according to claim 6, further comprising:
a regression device connecting the second arm (6) configured to support the table (20) and the main body (10), and moving the table (20),
wherein the regression device is configured to move the table (20) to a fourth position based on an angle of the table rotated by an external force and a preset angle.

12. The ultrasound diagnostic apparatus according to claim 11, wherein the regression device is configured to move the table (20) to a reference position when the angle of the table rotated by the external force exceeds a predetermined angle.

## Patentansprüche

1. Ultraschalldiagnosevorrichtung, die Folgendes umfasst:
eine Tisch (20), der zum Montieren mindestens einer Sonde (P) konfiguriert ist;
einen ersten Sensor (21), der zum Erfassen einer Typinformation der Sonde und eines Montagezustands der Sonde konfiguriert ist;
eine Antriebseinrichtung (40), die zum Erzeugen einer Antriebskraft zum Bewegen des Tisches konfiguriert ist; und
eine Steuerung (100),
**GEKENNZEICHNET DURCH**
einen zweiten Sensor (50), der zum Erfassen einer Position des Tisches konfiguriert ist;
einen dritten Sensor (35), der zum Erfassen einer Geste des Benutzers konfiguriert ist; und
wobei die Steuerung (100) dazu konfiguriert ist, die Antriebseinrichtung (40) basierend auf Erfassungsergebnissen des ersten Sensors und des zweiten Sensors zu steuern, und eine erste Position des Tisches (20) basierend auf einem Erfassungsergebnis des zweiten Sensors (50) zu bestimmen und den Tisch (20) von der ersten Position in eine zweite Position zu bewegen basierend auf einem Erfassungsergebnis des dritten Sensors (35).

2. Ultraschalldiagnosevorrichtung nach Anspruch 1, die weiterhin Folgendes umfasst:
eine Anzeige (31), die dazu konfiguriert ist, ein Ultraschallbild und eine Schnittstelle auszugeben,
wobei die Steuerung (100) dazu konfiguriert ist, zu bestimmen, ob der Montagezustand der auf dem Tisch (20) montierten Sonde (P) geändert wurde, basierend auf einem Erfassungsergebnis des ersten Sensors (21), und den Montagezustand und eine Typinformation der Sonde, deren Montagezustand geändert wurde, auf der Anzeige (31) anzuzeigen.

3. Ultraschalldiagnosevorrichtung nach Anspruch 2, wobei die Steuerung (100) dazu konfiguriert ist, die Bewegung des Tisches (20) für eine voreingestellte Zeit zu stoppen, nachdem der Montagezustand der Sonde (P) geändert wurde, und den Tisch (20) von der zweiten Position in eine dritte Position zu bewegen basierend auf der voreingestellten Zeit und dem Erfassungsergebnis des ersten Sensors (21).

4. Ultraschalldiagnosevorrichtung nach Anspruch 1, wobei die Steuerung (100) dazu konfiguriert ist, die Position des Tisches (20) durch den zweiten Sensor (50) zu bestimmen basierend auf einem Benutzereingabebefehl bezüglich des Endes der Bildgebungssequenz, und den Tisch (20) von der bestimmten Position des Tisches zu einer Referenzposition zu bewegen.

5. Ultraschalldiagnosevorrichtung nach Anspruch 1, wobei der erste Sensor (21) mindestens eine in dem Tisch (20) vorgesehene Sondenhalterung oder einen Magnetsensor, einen Metallsensor und/oder einen HF-Sensor, der innerhalb des Tisches vorgesehen ist, umfasst.

6. Ultraschalldiagnosevorrichtung nach Anspruch 1, die weiterhin Folgendes umfasst:
einen Hauptkörper (10) mit einem Strahlformer (80) und einem Verbinder (70), der mit der mindestens einen Sonde (P) verbunden ist;
einen ersten Arm (4), der dazu konfiguriert ist, eine Eingabevorrichtung zu tragen, die konfiguriert ist, um einen Benutzereingabebefehl zu empfangen, und an einer Seite des Hauptkörpers angelenkt ist, um drehbar zu sein; und
einen zweiten Arm (6), der dazu konfiguriert ist, den Tisch (20) zu tragen, an der anderen Seite des Hauptkörpers angelenkt und in Bezug auf eine erste Referenzachse und eine zweite Referenzachse drehbar gekoppelt ist,
wobei die Steuerung (100) dazu konfiguriert ist, den Drehradius des zweiten Arms (6) durch Einstellen der Antriebseinrichtung (40) einzustellen.

7. Ultraschalldiagnosevorrichtung nach Anspruch 6, wobei der zweite Sensor (50) in dem Hauptkörper (10) vorgesehen ist und einen Hallsensor und/oder einen Motorpositionssensor umfasst,
wobei der dritte Sensor (35) innerhalb des Tisches (20) oder auf der Eingavorrichtung vorgesehen ist, die konfiguriert ist, um einen Benutzereingabebefehl zu empfangen, und ein Kameramodul und/oder einen Ultraschallsensor umfasst.

8. Steuerverfahren einer Ultraschalldiagnosevorrichtung mit einem Tisch (20), der zum Montieren mindestens einer Sonde (P) konfiguriert ist, wobei das Verfahren Folgendes umfasst:
Erfassen einer Typinformation der Sonde und eines Montagezustands der Sonde (P) durch einen ersten Sensor (21);
Erfassen einer Position des Tisches (20) durch einen zweiten Sensor (50);
Erfassen einer Geste eines Benutzers durch einen dritten Sensor (35);
Bestimmen einer ersten Position des Tisches (20) basierend auf einem Erfassungsergebnis des zweiten Sensors (50); und
Bewegen des Tisches (20) basierend auf einer Änderung des erfassten Montagezustands und einer Position des Tisches,
wobei das Bewegen Folgendes umfasst:
Bewegen des Tisches (20) von der ersten Position in eine zweite Position basierend auf der Geste des Benutzers.

9. Steuerungsverfahren nach Anspruch 8, das weiterhin Folgendes umfasst:
Bestimmen, ob der Montagezustand der Sonde (P) geändert wurde, nachdem der Tisch (20) in die zweite Position bewegt wurde; und
Anzeigen des Montagezustands und einer Typinformation der Sonde (P), deren Montagezustand geändert wurde, auf einer Anzeige (31).

10. Steuerungsverfahren nach Anspruch 8, das weiterhin Folgendes umfasst:
Stoppen der Bewegung des Tisches (20) für eine voreingestellte Zeit, nachdem der Montagezustand der Sonde (P) geändert wurde,
wobei das Bewegen Folgendes umfasst:
Bewegen des Tisches (20) von der zweiten Position in eine dritte Position basierend auf der voreingestellten Zeit und einer Änderung des Montagezustands der Sonde (P).

11. Ultraschalldiagnosevorrichtung nach Anspruch 6, die weiterhin Folgendes umfasst:
eine Regressionsvorrichtung, die den zweiten Arm (6), der konfiguriert ist, um den Tisch (20) zu tragen, mit dem Hauptkörper (10) verbindet und den Tisch (20) bewegt,
wobei die Regressionsvorrichtung dazu konfiguriert ist, den Tisch (20) in eine vierte Position zu bewegen basierend auf einem Winkel des Tisches, der durch eine externe Kraft gedreht wird, und einem voreingestellten Winkel.

12. Ultraschalldiagnosevorrichtung nach Anspruch 11, wobei die Regressionsvorrichtung konfiguriert ist, um den Tisch (20) zu einer Referenzposition zu bewegen, wenn der Winkel des Tisches, der durch die externe Kraft gedreht wird, einen vorbestimmten Winkel überschreitet.

## Revendications

1. Appareil d'échographie diagnostique comprenant :
une table (20) conçue pour le montage d'au moins une sonde (P),
un premier détecteur (21) conçu pour détecter une information relative au type de la sonde et un état de montage de la sonde,
un organe d'entraînement (40) conçu pour générer une force d'entraînement permettant d'actionner la table, et
un organe de commande (100) ;
**CARACTÉRISÉ PAR**
un deuxième détecteur (50) conçu pour détecter la position de la table, et
un troisième détecteur (35) conçu pour détecter un geste de l'utilisateur ;
ledit organe de commande (100) étant conçu pour commander l'organe d'entraînement (40) en fonction des résultats de détection du premier détecteur et du deuxième détecteur, et pour déterminer une première position de la table (20) en fonction du résultat de détection du deuxième détecteur (50) et pour actionner la table (20) de la première position à une deuxième position en fonction du résultat de détection du troisième détecteur (35).

2. Appareil d'échographie diagnostique selon la revendication 1, comprenant en outre :
un écran (31) conçu pour produire une image écho graphique et une interface,
ledit organe de commande (100) étant conçu pour déterminer si l'état de montage de la sonde (P) montée sur la table (20) a changé ou non en fonction du résultat de détection du premier détecteur (21) et pour afficher, sur l'écran (31), l'état de montage et une information relative au type de la sonde dont l'état de montage a changé.

3. Appareil d'échographie diagnostique selon la revendication 2, dans lequel l'organe de commande (100) est conçu pour arrêter d'actionner la table (20) pendant une durée prédéfinie après que l'état de montage de la sonde (P) a changé et pour actionner la table (20) de la deuxième position à une troisième position en fonction de la durée prédéfinie et du résultat de détection du premier détecteur (21).

4. Appareil d'échographie diagnostique selon la revendication 1, dans lequel l'organe de commande (100) est conçu pour déterminer la position de la table (20) au moyen du deuxième détecteur (50) en fonction d'une commande saisie par l'utilisateur concernant la fin de la séquence d'échographie et pour actionner la table (20) de ladite position déterminée de la table à une position de référence.

5. Appareil d'échographie diagnostique selon la revendication 1, dans lequel le premier détecteur (21) comprend au moins une monture pour sonde prévue dans la table (20) ou un détecteur magnétique, un détecteur de métal et/ou un détecteur RF prévus à l'intérieur de la table.

6. Appareil d'échographie diagnostique selon la revendication 1, comprenant en outre :
un corps principal (10) comprenant un dispositif de formation de faisceaux (80) et un connecteur (70) connecté à l'au moins une sonde (P),
un premier bras (4) qui est conçu pour supporter un organe de saisie conçu pour recevoir une commande saisie par l'utilisateur et qui est monté articulé sur un côté du corps principal afin d'être rotatif, et
un deuxième bras (6) qui est conçu pour supporter la table (20) et qui est monté articulé sur l'autre côté du corps principal en étant couplé rotatif par rapport à un premier axe de référence et un deuxième axe de référence ;
ledit organe de commande (100) étant conçu pour régler le rayon de rotation du deuxième bras (6) en réglant l'organe d'entraînement (40).

7. Appareil d'échographie diagnostique selon la revendication 6, dans lequel le deuxième détecteur (50) est prévu dans le corps principal (10) et comprend un capteur à effet Hall et/ou un capteur de position de moteur, et
dans lequel le troisième détecteur (35) est prévu à l'intérieur de la table (20) ou sur l'organe de saisie conçu pour recevoir une commande saisie par l'utilisateur et comprend un module de caméra et/ou un détecteur d'ultrasons.

8. Procédé de commande d'un appareil d'échographie diagnostique comprenant une table (20) conçue pour le montage d'au moins une sonde (P), comprenant ;
la détection d'une information relative au type de la sonde et d'un état de montage de la sonde (P) par un premier détecteur (21) ;
la détection de la position de la table (20) par un deuxième détecteur (50) ;
la détection d'un geste d'un utilisateur par un troisième détecteur (35) ;
la détermination d'une première position de la table (20) en fonction du résultat de détection du deuxième détecteur (50) ; et
l'actionnement de la table (20) en fonction d'un changement concernant l'état de montage détecté et la position de la table,
ledit actionnement comprenant :
l'actionnement de la table (20) de la première position à une deuxième position en fonction du geste de l'utilisateur.

9. Procédé de commande selon la revendication 8, comprenant en outre :
la détermination du fait que l'état de montage de la sonde (P) a changé ou non après que la table (20) a été actionnée pour prendre la deuxième position, et
l'affichage, sur un écran (31), de l'état de montage et d'une information relative au type de la sonde (P) dont l'état de montage a changé.

10. Procédé de commande selon la revendication 8, comprenant en outre :
l'arrêt de l'actionnement de la table (20) pendant une durée prédéfinie après que l'état de montage de la sonde (P) a changé,
ledit actionnement comprenant :
l'actionnement de la table (20) de la deuxième position à une troisième position en fonction de la durée prédéfinie et d'un changement concernant l'état de montage de la sonde (P).

11. Appareil d'échographie diagnostique selon la revendication 6, comprenant en outre :
un dispositif de rappel reliant le deuxième bras (6), conçu pour supporter la table (20), au corps principal (10), et actionnant la table (20),
ledit dispositif de rappel étant conçu pour actionner la table (20) vers une quatrième position en fonction d'un angle de la table ayant subi une rotation sous l'effet d'une force externe, et d'un angle prédéfini.

12. Appareil d'échographie diagnostique selon la revendication 11, dans lequel le dispositif de rappel est conçu pour actionner la table (20) vers une position de référence lorsque l'angle de la table ayant subi une rotation sous l'effet de la force externe a dépassé un angle prédéterminé.
